# EUROPEAN PATENT SPECIFICATION

(11) **EP 3 220 375 B1**
(45) Date of publication and mention of the grant of the patent: **08.01.2020**
(21) Application number: 16205654.3
(22) Date of filing: 21.12.2016
(51) Int. Cl.: A61F 2/82, A61F 2/958, A61F 2/95, G09B 23/28, G09B 23/30, G09B 23/34

(54) **TRAINING DEVICE AND TRAINING DEVICE SYSTEM**
TRAININGSVORRICHTUNG UND TRAININGSVORRICHTUNGSSYSTEM
DISPOSITIF D'ENTRAÎNEMENT ET SYSTÈME DE DISPOSITIF D'ENTRAÎNEMENT

(30) Priority: 16.03.2016 JP 2016052790
(43) Date of publication of application: 20.09.2017
(73) Proprietor: Takashima Sangyo Co. Ltd., Chino-shi, Nagano 391-0012 (JP); OSAKA UNIVERSITY, Suita-shi, Osaka 565-0871 (JP)
(72) Inventor: OKAYAMA, Keita, Suita-shi, Osaka 565-0871 (JP); SAKATA, Yasushi, Suita-shi, Osaka 565-0871 (JP); YANAGISHIMA, Keigo, Chino-shi, Nagano 391-0012 (JP); AONUMA, Shigeru, Chino-shi, Nagano 391-0012 (JP); SATOU, You, Chino-shi, Nagano 391-0012 (JP); YAZAKI, Kouhei, Chino-shi, Nagano 391-0012 (JP); OOWA, Junji, Chino-shi, Nagano 391-0012 (JP)
(74) Representative: Schmitt-Nilson Schraud Waibel Wohlfrom Patentanwälte Partnerschaft mbB

(56) References cited:
- EP-A1- 0 433 447
- WO-A2-01/97740
- US-A- 5 242 451

## Description

### BACKGROUND OF THE INVENTION

### Field of the Invention

The present invention relates to a training device to be inserted and placed inside a three-dimensional model of a biological organ manufactured for training. The present invention also relates to a training device system including the training device.

### Background

Hitherto, a simulation system for placing a stent graft in an aorta model simulating the aorta of a patient is known (see, for example, Japanese Patent Application Laid-open No. 2015-64487). In the simulation system described in Japanese Patent Application Laid-open No. 2015-64487, a stent graft mounted on a catheter in a compressed state is inserted through a tubular body connected to the aorta model. When the stent graft mounted on the catheter reaches an affected area in the aorta model, the stent graft is expanded and the catheter is removed, so that the stent graft is placed in the affected area. For example, the simulation system is used by doctors or medical students for training of stent grafting.

In the simulation system described in Japanese Patent Application Laid-open No. 2015-64487, when the stent graft reaches an affected area in the aorta model, the stent graft is expanded to be placed in the affected area, and it is therefore difficult to retrieve the stent graft expanded in the affected area from the aorta model. In other words, in the simulation system, once the training of stent grafting is performed, it is difficult to reuse the stent graft used for the training. In general, a stent graft used for the training of stent grafting is expensive. Thus, the cost of the conventional training of stent grafting is high.

US 5 242 451 A discloses a stent to be inserted and placed inside a biological organ, under a state in which the stent is mounted on a balloon of a balloon catheter, wherein the stent is a cylindrical body formed of a shape-memory alloy, the stent is expandable under a room temperature enviroment, and the stent is contracted when heated to a predetermined temperature higher than room temperature, until an inner diameter of the stent becomes smaller than outer diameter of the balloon in a contracted state, and returns to an original shape thereof.

WO 01/97740 A2 discloses a balloon catheter which is provided with a series of inflatable ribs in order to provide an anchoring force when arranged in position in for instance an aorta.

### SUMMARY OF THE INVENTION

It is an object of the present invention to provide a training device to be inserted and placed inside a three-dimensional model of a biological organ manufactured for training, which is capable of reducing the cost of training in which the training device is inserted and placed inside the three-dimensional model. It is another object of the present invention to provide a training device system including the training device.

The object is achieved by the training device claimed in claim 1, and by the training device system claimed in claim 4. Embodiments of the invention are claimed in the respective dependent claims.

In order to achieve the object, the present invention provides a training device to be inserted and placed inside a three-dimensional model of a biological organ manufactured for training, under a state in which the training device is mounted on a balloon of a balloon catheter. The training device is a cylindrical stent formed of a shape-memory alloy. The stent is expandable under a room temperature environment. The stent is contracted when heated to a predetermined temperature higher than room temperature, until an inner diameter of the stent becomes smaller than an outer diameter of the balloon in a contracted state, and returns to an original shape thereof.

Furthermore, in order to achieve the object, the present invention provides a training device to be inserted and placed inside a three-dimensional model of a biological organ manufactured for training, under a state in which the training device is mounted on a balloon of a balloon catheter. The training device includes a cylindrical stent formed of a shape-memory alloy. The stent is expandable under a room temperature environment. The stent is contracted when heated to a predetermined temperature higher than room temperature, until an inner diameter of the stent becomes smaller than an outer diameter of the balloon in a contracted state, and returns to an original shape thereof.

According to the present invention, the stent that is the training device or the stent that constitutes a part of the training device is formed of a shape-memory alloy. According to the present invention, the stent is expandable under a room temperature environment, and when heated to a predetermined temperature higher than room temperature, the stent is contracted to return to the original shape thereof. Thus, according to the present invention, after the stent in the contracted state is inserted inside the three-dimensional model of the biological organ manufactured for training and is thereafter expanded under a room temperature environment by the balloon of the balloon catheter and placed inside the three-dimensional model, when heated to a predetermined temperature higher than room temperature, the stent is contracted to return to its original shape. Consequently, according to the present invention, by heating and contracting the stent placed inside the three-dimensional model, the training device placed inside the three-dimensional model can be removed from inside the three-dimensional model. In other words, according to the present invention, the training device placed inside the three-dimensional model can be retrieved from inside the three-dimensional model.

According to the present invention, when the stent is heated to a predetermined temperature, the stent is contracted until the inner diameter of the stent becomes smaller than the outer diameter of the balloon in the contracted state. Thus, after the training device is retrieved from inside the three-dimensional model, by heating the stent to a predetermined temperature under the state in which the contracted balloon is inserted in an inner circumferential side of the stent expanded under a room temperature environment, the training device can be mounted on the balloon of the balloon catheter. In other words, according to the present invention, the training device retrieved from inside the three-dimensional model can be mounted on the balloon of the balloon catheter again.

As described above, according to the present invention, the training device placed inside the three-dimensional model can be retrieved from inside the three-dimensional model, and the training device retrieved from inside the three-dimensional model can be mounted on the balloon of the balloon catheter again. Thus, according to the present invention, the retrieved training device can be repeatedly used for training in which the training device is inserted and placed inside the three-dimensional model for training. Consequently, the present invention can reduce the cost of training in which the training device is inserted and placed inside the three-dimensional model.

According to the present invention, when the stent is heated to a predetermined temperature, the stent is contracted until the inner diameter of the stent becomes smaller than the outer diameter of the balloon in the contracted state, and hence the training device can be easily mounted on the balloon.

In the present invention, the stent has a retaining portion configured to prevent the stent in the contracted state from falling off from the balloon in the contracted state. For example, the retaining portion is at least one of a rough surface portion formed on an inner circumferential surface of the stent, a protruding portion that protrudes to an inner circumferential side of the stent, and a small-diameter portion that is formed at an end portion of the stent and has an inner diameter smaller than at a central part of the stent. This configuration can prevent the training device from falling off from the balloon that is inserted inside the three-dimensional model under the contracted state. Consequently, even when the internal shape of the three-dimensional model is complicated, the training device can be reliably inserted to a certain position inside the three-dimensional model.

In the present invention, it is preferred that the stent have a hook portion configured to catch a distal end portion of a removal instrument for removing the training device placed inside the three-dimensional model. With this configuration, even when the internal shape of the three-dimensional model is complicated, the training device in the contracted state placed inside the three-dimensional model can be easily removed from inside the three-dimensional model by using the removal instrument.

The training device of the present invention may be used for a training device system including the balloon catheter. The training device system can reduce the cost of training in which the training device is inserted and placed inside a three-dimensional model.

In the present invention, it is preferred that the balloon have a protruding portion formed or fixed on an outer circumferential surface thereof, the protruding portion being arranged on both sides or one side of the stent and configured to prevent the stent in a contracted state from falling off from the balloon in a contracted state. This configuration can prevent the training device from falling off from the balloon that is inserted inside the three-dimensional model under the contracted state. Consequently, even when the internal shape of the three-dimensional model is complicated, the training device can be reliably inserted to a certain position inside the three-dimensional model.

In the present invention, for example, the training device system may include a cylindrical mounting jig for mounting the training device on the balloon. The stent in the expanded state may be arrangeable on an inner peripheral side of the mounting jig. The mounting jig may include a contracting portion configured to contract the stent by performing at least one of pressurization and heating of the stent in the expanded state arranged on the inner peripheral side of the mounting jig. When the contracting portion contracts the stent under a state in which the stent in the expanded state is arranged on the inner peripheral side of the mounting jig and the balloon in the contracted state is inserted in an inner circumferential side of the stent, the training device may be mounted on the balloon. In this case, the training device can be mounted on the balloon relatively easily by using the mounting jig.

In the present invention, for example, the training device system may further include: a mandrel to be inserted in an inner circumferential side of the stent in order to expand the stent under a room temperature environment; and a substantially cylindrical expanding jig that is used together with the mandrel in order to expand the stent under a room temperature environment and in which the stent is arranged on an inner circumferential side thereof. The expanding jig may include: a cylindrical first cylinder portion that constitutes one end-side part of the expanding jig; and a cylindrical second cylinder portion that constitutes another end-side part of the expanding jig and that is arranged coaxially with the first cylinder portion. The first cylinder portion may have an inner diameter that is substantially equal to an outer diameter of the stent expanded by the mandrel, or larger than the outer diameter of the stent expanded by the mandrel. The second cylinder portion may have an inner diameter that is smaller than the outer diameter of the stent expanded by the mandrel. The stent mounted on the mandrel may be inserted in an inner circumferential side of the first cylinder portion from one end side of the expanding jig. In this case, the training device can be mounted on the balloon relatively easily by using the expanding jig.

As described above, the present invention can reduce the cost of training in which a training device is inserted and placed inside a three-dimensional model.

### BRIEF DESCRIPTION OF THE DRAWINGS

Figs. 1A and 1B are side views of a training device according to the present invention.
Fig. 2 is a side view of a balloon catheter on which the training device illustrated in Figs. 1A and 1B is mounted.
Figs. 3A to 3F are views for illustrating a procedure of training in which the training device illustrated in Figs. 1A and 1B is inserted and placed inside a three-dimensional model.
Fig. 4 is a side view of a training device according to an embodiment of the present invention.
Figs. 5A and 5B are views of the training device according to another embodiment of the present invention, in which Fig. 5A is a perspective view of an end portion of the training device and Fig. 5B is a front view of the training device.
Figs. 6A and 6B are views of a training device according to still another embodiment of the present invention, in which Fig. 6A is a front view of the training device and Fig. 6B is a perspective view of an end portion of the training device.
Fig. 7 is a side view of a balloon catheter according to further another embodiment of the present invention.
Figs. 8A and 8B are sectional views for illustrating a structure of a mounting jig for mounting the training device illustrated in Figs. 1A and 1B on a balloon.
Figs. 9A to 9D are sectional views for illustrating a structure of an expanding jig for expanding the stent illustrated in Figs. 1A and 1B.

### DETAILED DESCRIPTION OF THE PREFERRED EMBODIMENTS

Referring to the accompanying drawings, embodiments of the present invention are described below.

### Structure of Training Device and Procedure of Training Using Training Device

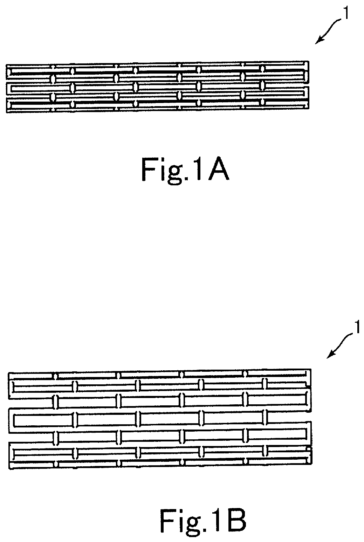

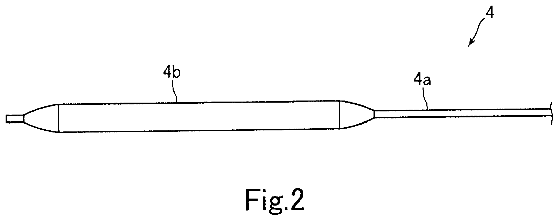

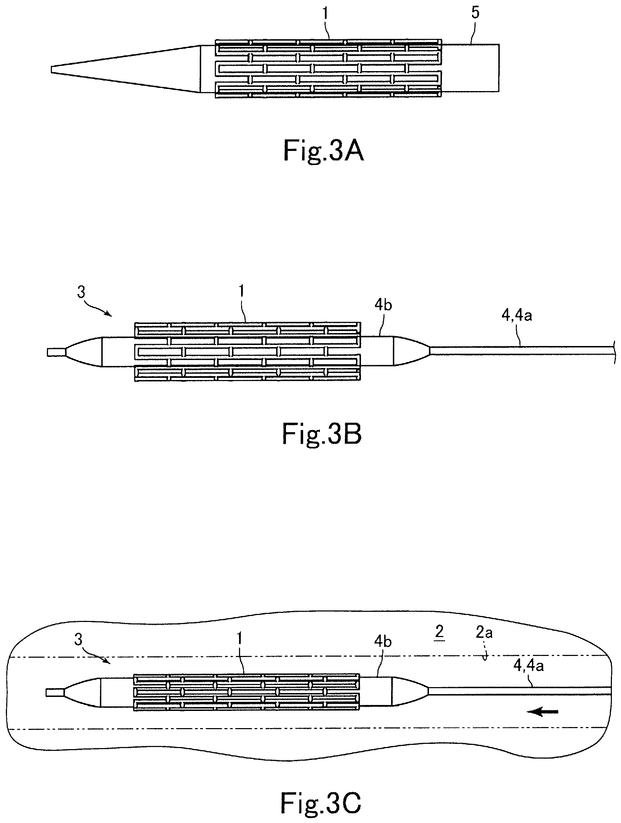

Figs. 1A and 1B are side views of a training device 1 of the present invention. Fig. 2 is a side view of a balloon catheter 4 on which the training device 1 illustrated in Figs. 1A and 1B is mounted. Figs. 3A to 3F are views for illustrating a procedure of training in which the training device 1 illustrated in Figs. 1A and 1B is inserted and placed inside a three-dimensional model 2. The training device 1 includes a retaining portion (not shown in Figs. 1A, 1B, and 3A to 3F) which will be described below.

The training device 1 is a device for use in training in which the training device 1 is inserted and placed inside the three-dimensional model 2 of a biological organ manufactured for training. The training device 1 in the present embodiment is a stent formed into a cylindrical shape. A tube portion 2a that simulates a blood vessel of a patient is formed inside the three-dimensional model 2. The stent is used for training in which the stent is inserted and placed in the tube portion 2a. The training device 1 in the present embodiment is hereinafter referred to as a "stent 1".

The three-dimensional model 2 is formed of, for example, a transparent silicone resin (silicone). The stent 1 is arranged on the inner side of a tubular part in a human body, such as blood vessels, trachea, large intestine, or bile ducts, and used to expand the tubular member from inside. The stent 1 is formed into a substantially cylindrical shape as a whole. The side surface of the stent 1 is formed into a net. Note that the stent 1 may be formed into a substantially cylindrical shape as a whole and also formed into a spiral shape.

The stent 1 is formed of a shape-memory alloy, such as a nickel-titanium alloy. In the present embodiment, the stent 1 is plastically deformable under a room temperature environment, and the stent 1 is expandable under a room temperature environment. The stent 1 is contracted when heated to a predetermined temperature higher than room temperature, and returns to its original shape. Specifically, when the stent 1 that has been expanded under a room temperature environment as illustrated in Fig. 1B is heated to a predetermined temperature, the stent 1 is contracted to return to its original shape as illustrated in Fig. 1A. For example, the expanded stent 1 is contracted to return to its original shape when the temperature becomes 50°C or more.

The stent 1 constitutes a part of a training device system 3. The training device system 3 includes the balloon catheter 4 and a mandrel 5. The balloon catheter 4 is a commercially available product, and is formed into a flexible thin tube. The balloon catheter 4 includes a catheter tube 4a and a balloon 4b attached on a distal end side of the catheter tube 4a. For training, the stent 1 is inserted and placed in the tube portion 2a inside the three-dimensional model 2 under a state in which the stent 1 is mounted on the balloon 4b of the balloon catheter 4. Note that the balloon catheter 4 may be dedicated for training in which the stent 1 is inserted and placed in the tube portion 2a of the three-dimensional model 2.

Training using the stent 1 is performed as follows. First, as illustrated in Fig. 3A, under a room temperature environment, the mandrel 5 is inserted in the inner circumferential side of the stent 1 that has been contracted and returned to its original shape, so that the stent 1 is expanded. An indeflator (compression/decompression device for balloon catheter) is used to contract the balloon 4b of the balloon catheter 4, and then the contracted balloon 4b is inserted in the inner circumferential side of the expanded stent 1 as illustrated in Fig. 3B.

Next, liquid, such as water, normal saline, or a contrast agent, is poured into the balloon catheter 4 with the indeflator and the balloon 4b is expanded (increased in diameter) so that an inner circumferential surface of the stent 1 and an outer circumferential surface of the balloon 4b are brought into intimate contact with each other. Although the stent 1 has already been expanded by the mandrel 5, it is desired to sufficiently expand the balloon 4b so that the stent 1 is further expanded in order to bring the inner circumferential surface of the stent 1 and the outer circumferential surface of the balloon 4b into intimate contact with each other.

Then, the stent 1 in the expanded state, which is in intimate contact with the outer circumferential surface of the balloon 4b in the expanded state, is immersed in heated liquid, such as water or alcohol, and heated to a predetermined temperature equal to or higher than room temperature, whereby the stent 1 is contracted as illustrated in Fig. 3C. In this case, the stent 1 is heated and contracted while the indeflator is discharging the liquid from the balloon catheter 4.

When the stent 1 in the present embodiment is heated to a predetermined temperature equal to or higher than room temperature, the stent 1 is contracted until the inner diameter of the stent 1 becomes smaller than the outer diameter of the balloon 4b in the contracted state (specifically, the outer diameter of the balloon 4b contracted most by the indeflator). Thus, when the stent 1 heated in the liquid reaches the predetermined temperature, the stent 1 is contracted to be mounted on the balloon 4b. When the stent 1 is contracted to be mounted on the balloon 4b, the stent 1 and the balloon 4b in the liquid are removed from the liquid. Note that, in the present embodiment, when the stent 1 in the expanded state in intimate contact with the outer circumferential surface of the balloon 4b is immersed in the heated liquid, the temperature of the liquid in the balloon catheter 4 is increased, and hence the stent 1 can be efficiently heated and contracted.

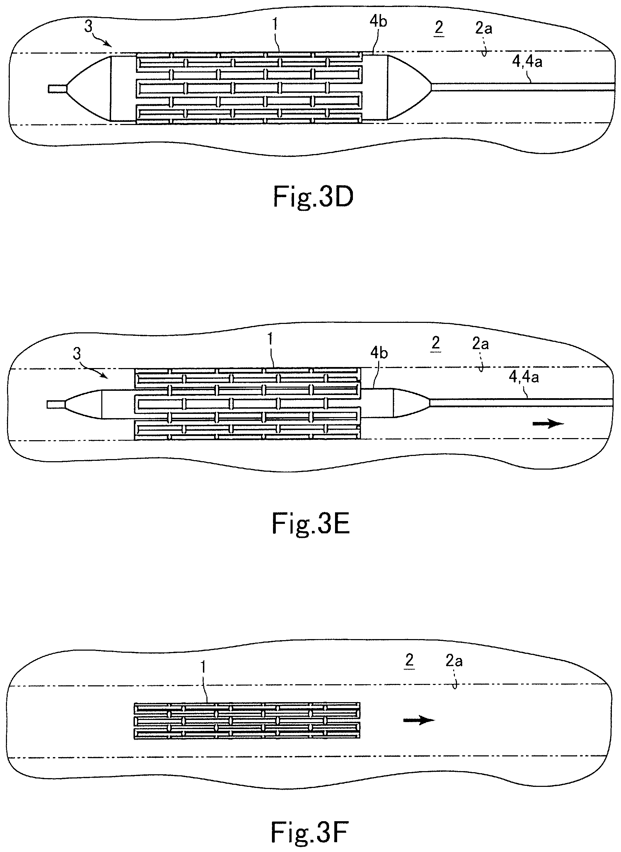

Next, the stent 1 mounted on the balloon 4b is inserted in the tube portion 2a of the three-dimensional model 2 (see Fig. 3C). Then, as illustrated in Fig. 3D, under a room temperature environment, the balloon 4b is expanded by the indeflator to expand the stent 1 so that the stent 1 is placed in the tube portion 2a. Then, as illustrated in Fig. 3E, the balloon 4b is contracted by the indeflator, and the balloon catheter 4 is removed from the tube portion 2a. When the balloon catheter 4 is removed from the tube portion 2a, the stent 1 is placed in the tube portion 2a. When the stent 1 is placed in the tube portion 2a, the training using the stent 1 is finished.

When the training using the stent 1 is finished, the stent 1 placed in the tube portion 2a is heated to a predetermined temperature and contracted to return to its original state (see Fig. 3F). For example, the stent 1 is immersed in heated liquid together with the three-dimensional model 2, and the stent 1 is heated and contracted to return to its original state. In the case where it is difficult to directly heat the stent 1, such as when the tube portion 2a is formed to simulate a coronary artery of the heart, a device such as a catheter may be used to deliver heated liquid to a site concerned in the tube portion 2a, thereby heating and contracting the stent 1. When the stent 1 returns to its original state, the stent 1 can be removed from the tube portion 2a, and hence the stent 1 is removed and retrieved from the tube portion 2a. For example, forceps are used to remove and retrieve the stent 1 from the tube portion 2a.

### Main Effects of using a shape Memory Alloy

As described above, in the present invention, the stent 1 is formed of a shape-memory alloy. It also comprises a retaining portion (not shown in Figures 1A, 1B, and 3A to 3F). When the stent 1 that has been inserted in the tube portion 2a and then expanded by the balloon 4b under a room temperature environment and placed in the tube portion 2a is heated to a predetermined temperature, the stent 1 is contracted to return to its original shape. Thus, in the present invention, as described above, the stent 1 placed in the tube portion 2a can be retrieved from the tube portion 2a after training by heating and contracting the stent 1. In the present invention, when the stent 1 is heated to a predetermined temperature, the stent 1 is contracted until the inner diameter of the stent 1 becomes smaller than the outer diameter of the balloon 4b in the contracted state, and hence after the stent 1 is retrieved from the tube portion 2a, the stent 1 is mounted on the balloon 4b by heating the stent 1 to a predetermined temperature under the state in which the contracted balloon 4b is inserted in the inner circumferential side of the stent 1 that has been expanded under a room temperature environment. In other words, in the present invention, the stent 1 retrieved from the tube portion 2a can be mounted on the balloon 4b again.

As described above, in the present invention, the stent 1 placed in the tube portion 2a of the three-dimensional model 2 can be retrieved from the tube portion 2a, and the retrieved stent 1 can be mounted on the balloon 4b again. Thus, in the present invention, the retrieved stent 1 can be repeatedly used for training in which the stent 1 is inserted and placed in the tube portion 2a of the three-dimensional model 2. Consequently, in the present invention, the cost of training in which the stent 1 is inserted and placed inside the three-dimensional model 2 can be reduced.

In the present invention, when the stent 1 is heated to a predetermined temperature, the stent 1 is contracted until the inner diameter of the stent 1 becomes smaller than the outer diameter of the balloon 4b in the contracted state. Consequently, the stent 1 can be easily mounted on the balloon 4b.

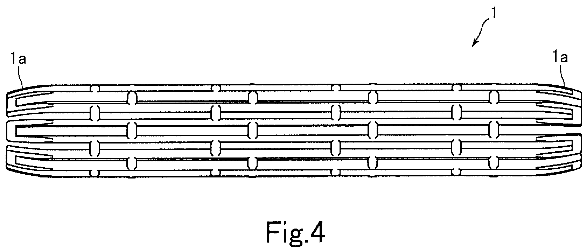

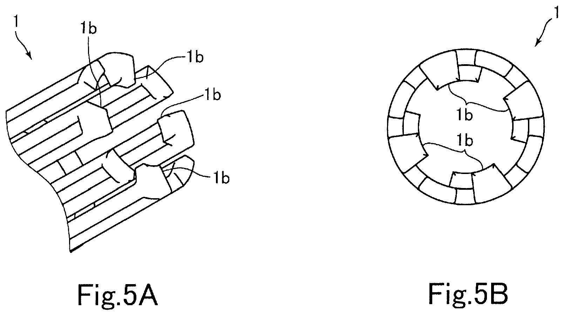

Fig. 4 is a side view of a stent 1 according to an embodiment of the present invention. Figs. 5A and 5B are views of the stent 1 according to another embodiment of the present invention, in which Fig. 5A is a perspective view of an end portion of the stent 1 and Fig. 5B is a front view of the stent 1.

In the embodiments of the invention, a retaining portion configured to prevent the contracted stent 1 from falling off from the contracted balloon 4b is formed on the stent 1. The retaining portion formed on the stent 1 is, for example, a rough surface portion formed by roughing the whole or part of the inner circumferential surface of the stent 1. The surface roughness of the rough surface portion is set so that the contracted stent 1 that is inserted in the tube portion 2a under a state in which the stent 1 mounted on the balloon 4b is not displaced from the contracted balloon 4b as illustrated in Fig. 3C.

The retaining portion formed on the stent 1 is, for example, a small-diameter portion 1a that is formed at an end portion of the stent 1 and whose inner diameter and outer diameter are smaller than the central part of the stent 1 as illustrated in Fig. 4. The small-diameter portions 1a are formed at both end portions of the stent 1. The inner diameter of the small-diameter portion 1a is set so that the contracted stent 1 that is inserted in the tube portion 2a under a state in which the stent 1 is mounted on the balloon 4b is not displaced from the contracted balloon 4b as illustrated in Fig. 3C. Note that the small-diameter portion 1a may be formed only at one end portion of the stent 1.

The retaining portion formed on the stent 1 may be a protruding portion 1b that protrudes on the inner circumferential side of the stent 1 as illustrated in Figs. 5A and 5B. The protruding portions 1b are formed at both end portions or one end portion of the stent 1. The protruding portions 1b are formed at a plurality of locations at constant pitches in the circumferential direction of the stent 1 as illustrated in Fig. 5B as viewed in the axial direction of the stent 1 formed into a substantially cylindrical shape. The protruding amount of the protruding portion 1b to the inner circumferential side of the stent 1 is set so that the contracted stent 1 that is inserted in the tube portion 2a under a state in which the stent 1 is mounted on the balloon 4b is not displaced from the contracted balloon 4b as illustrated in Fig. 3C. Note that the protruding portions 1b may be formed at the central part of the stent 1.

The stent 1 may include the rough surface portion formed on the inner circumferential surface of the stent 1 and the small-diameter portion 1a. Alternatively, the stent 1 may include the rough surface portion formed on the inner circumferential surface of the stent 1 and the protruding portion 1b. The stent 1 may include the small-diameter portion 1a and the protruding portion 1b. The stent 1 may include the rough surface portion formed on the inner circumferential surface of the stent 1, the small-diameter portion 1a, and the protruding portion 1b. Due to the retaining portion formed on the stent 1, the stent 1 can be prevented from falling off from the balloon 4b inserted in the tube portion 2a. Consequently, even when the tube portion 2a is formed to simulate a coronary artery and the shape of the tube portion 2a is complicated, the stent 1 can be reliably inserted to a certain position in the tube portion 2a.

### Modification of Stent

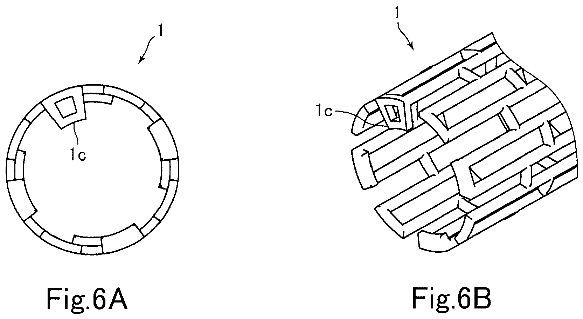

Figs. 6A and 6B are views of a stent 1 according to another embodiment of the present invention. Fig. 6A is a front view of the stent 1 and Fig. 6B is a perspective view of an end portion of the stent 1.

In the embodiment described above, a hook portion 1c to catch a distal end portion of a removal instrument (not shown) for removing the stent 1 placed in the tube portion 2a may be formed on the stent 1. The hook portion 1c is formed at one end portion of the stent 1 as illustrated in Figs. 6A and 6B. The hook portion 1c is formed into a flat plate that protrudes on the inner circumferential side of the stent 1. Specifically, the hook portion 1c is formed into a hook shape. A through hole passing through the hook portion 1c in the axial direction of the stent 1 is formed at the center of the hook portion 1c. In the case where the hook portion 1c is formed on the stent 1, for example, even when the tube portion 2a is formed to simulate a coronary artery and the shape of the tube portion 2a is complicated, the contracted stent 1 placed in the tube portion 2a can be easily removed from the tube portion 2a by using the removal instrument.

Note that the hook portion 1c may be a hook-shaped protrusion or a flat plate-shaped protrusion that protrudes in the axial direction of the stent 1. A hook portion formed separately from the stent 1 may be attached to the stent 1. The hook portion in this case is formed of, for example, threads, a threadlike resin, or a thin film resin. The hook portion in this case may be formed into a cord shape, but it is preferred that the hook portion may be formed into a ring shape in order for the removal instrument to be easily hooked at the hook portion when the stent 1 is removed.

The removal instrument is, for example, a thin wire whose distal end is formed into a hook shape. Specifically, the removal instrument is a thin wire formed of a superelastic alloy and having a barb (protrusion pointed in the opposite direction) formed at its distal end. In this case, the stent 1 can be removed from the tube portion 2a under the state in which the barb at the distal end of the thin wire is hooked at the hook portion 1c of the stent 1 placed in the tube portion 2a. Consequently, even when the shape of the tube portion 2a is complicated, the stent 1 can be easily and reliably retrieved. The removal instrument may be a guide wire curved at its distal end, or forceps.

### Modification of Balloon Catheter

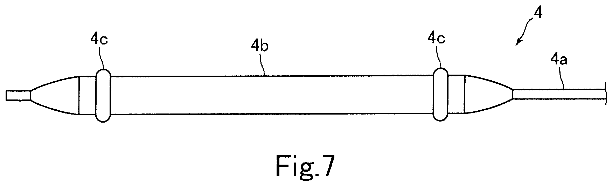

Fig. 7 is a side view of a balloon catheter 4 according to another embodiment of the present invention.

In the embodiment described above, a protruding portion 4c configured to prevent the contracted stent 1 from falling off from the contracted balloon 4b may be formed on the outer circumferential surface of the balloon 4b of the balloon catheter 4 as illustrated in Fig. 7. The protruding portions 4c are formed integrally with the balloon 4b. The protruding portions 4c are formed on both end sides of the balloon 4b so as to be arranged on both sides of the stent 1. The protruding portions 4c are formed into an annular shape along the circumferential direction of the outer circumferential surface of the balloon 4b, and protrude from the outer circumferential surface of the balloon 4b to the radially outer side of the balloon 4b. The protruding amount of the protruding portion 4c from the outer circumferential surface of the balloon 4b is set so that the contracted stent 1 that is inserted in the tube portion 2a under a state in which the stent 1 mounted on the balloon 4b is not displaced from the contracted balloon 4b as illustrated in Fig. 3C.

In the case where the protruding portions 4c are formed on the outer circumferential surface of the balloon 4b, the stent 1 can be prevented from falling off from the balloon 4b inserted into the tube portion 2a. Consequently, even when the shape of the tube portion 2a is complicated, the stent 1 can be reliably inserted to a certain position in the tube portion 2a. Note that the protruding portion 4c may be formed on one end side of the balloon 4b so as to be arranged on one side of the stent 1 (specifically, the rear side in the insertion direction of the stent 1). A protruding portion that is formed separately from the balloon 4b may be fixed to the balloon 4b. The protruding portion in this case is, for example, an O-ring or a metal boss. The protruding portion in this case may be a clip.

### First Modification of Training Device System

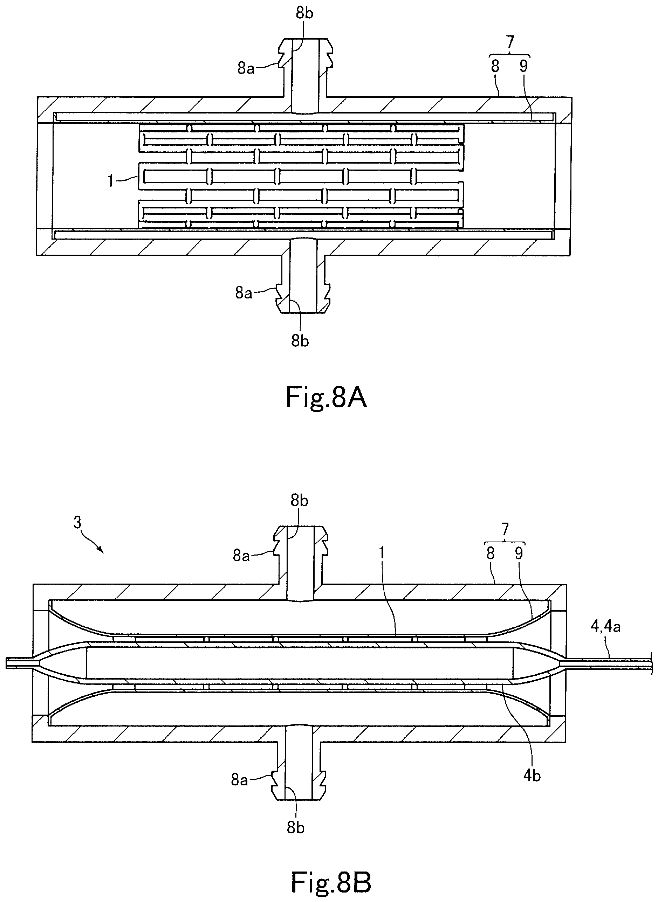

Figs. 8A and 8B are sectional views for illustrating a structure of a mounting jig 7 for mounting the stent 1 illustrated in Figs. 1A and 1B to the balloon 4b.

In the embodiment described above, the training device system 3 may include a mounting jig 7 for mounting the stent 1 on the balloon 4b. The mounting jig 7 is formed into a cylinder. Specifically, the mounting jig 7 is formed into a substantially cylindrical shape as a whole. The stent 1 in the expanded state can be arranged on the inner peripheral side of the mounting jig 7. In other words, the mounting jig 7 is formed to have such a size that enables the stent 1 in the expanded state to be arranged on the inner peripheral side of the mounting jig 7.

The mounting jig 7 includes a jig main body 8 formed into a substantially cylindrical shape and a cylindrical cylinder member 9 placed on the inner peripheral side of the jig main body 8. The cylinder member 9 is formed of, for example, an elastic member excellent in stretching property, such as rubber, and is formed separately from the jig main body 8. The cylinder member 9 is formed into a thin cylindrical shape. Both ends of the cylinder member 9 formed into a cylindrical shape are respectively fixed at both ends of the inner circumferential surface of the jig main body 8 in the axial direction of the jig main body 8.

In the jig main body 8, a tubular portion 8a that protrudes from the outer circumferential surface of the jig main body 8 to the radially outer side is formed or fixed. In the jig main body 8, a through hole 8b that passes from an outer circumferential end of the tubular portion 8a in the radial direction of the jig main body 8 to the inner circumferential surface of the jig main body 8 is formed. A supply source configured to supply heated fluid, such as warm water, is connected to the tubular portion 8a through a predetermined pipe, and the through hole 8b serves as a fluid passage through which the fluid passes.

When a heated fluid is supplied from the supply source so that the heated fluid flows between the inner circumferential surface of the jig main body 8 and the outer circumferential surface of the cylinder member 9 under the state in which the stent 1 in the expanded state through which the mandrel 5 is inserted in the inner circumferential side thereof is arranged on the inner circumferential side of the cylinder member 9 as illustrated in Fig. 8A and the balloon 4b in the contracted state is inserted in the inner circumferential side of the stent 1, the cylinder member 9 expands toward the inner peripheral side of the mounting jig 7 in a manner that the inner circumferential surface of the cylinder member 9 is brought into intimate contact with the outer circumferential surface of the stent 1. The stent 1 arranged on the inner circumferential side of the cylinder member 9 is heated by the fluid between the jig main body 8 and the cylinder member 9 to a predetermined temperature, and is contracted as illustrated in Fig. 8B. When the stent 1 is contracted, the stent 1 is mounted on the balloon 4b. When the stent 1 is mounted on the balloon 4b, the fluid between the jig main body 8 and the cylinder member 9 is discharged through the through hole 8b.

In the present modification, the fluid between the jig main body 8 and the cylinder member 9 and the cylinder member 9 correspond to a contracting portion configured to heat the stent 1 in the expanded state arranged on the inner peripheral side of the mounting jig 7 to contract the stent 1. When the contracting portion contracts the stent 1 under the state in which the stent 1 in the expanded state is arranged on the inner peripheral side of the mounting jig 7 and the balloon 4b in the contracted state is inserted in the inner circumferential side of the stent 1, the stent 1 is mounted on the balloon 4b. As described above, in the case where the training device system 3 includes the mounting jig 7, the stent 1 can be mounted on the balloon 4b relatively easily by using the mounting jig 7.

Note that a fluid flowing between the jig main body 8 and the cylinder member 9 may be an unheated roomtemperature fluid. In this case, the fluid flowing between the jig main body 8 and the cylinder member 9 is under high inflow pressure. In this case, when the fluid flows between the jig main body 8 and the cylinder member 9 so that the cylinder member 9 expands toward the inner peripheral side of the mounting jig 7, the stent 1 in the expanded state arranged on the inner peripheral side of the mounting jig 7 is pressurized toward the radially inner side by the cylinder member 9 that expands toward the inner peripheral side of the mounting jig 7, and is contracted so that the stent 1 is mounted on the balloon 4b. In this case, the cylinder member 9 and the fluid between the jig main body 8 and the cylinder member 9 serve as a contracting portion configured to contract the stent 1 by pressurizing the stent 1 in the expanded state arranged on the inner peripheral side of the mounting jig 7.

The mounting jig 7 may include, instead of the cylinder member 9, a substantially cylindrical contracting member arranged on the inner peripheral side of the jig main body 8. The contracting member is formed of a shape-memory alloy similarly to the stent 1, and is formed into a spiral shape, for example. The contracting member can be expanded under a room temperature environment. The contracting member is configured to contract to return to its original shape when heated to a predetermined temperature.

In this case, when the contracting member is heated to a predetermined temperature under a state in which the stent 1 in the expanded state is arranged on the inner circumferential side of the contracting member in the expanded state and the balloon 4b in the contracted state is inserted in the inner circumferential side of the stent 1, the contracting member is contracted in a manner that the inner circumferential surface of the contracting member is brought into intimate contact with the outer circumferential surface of the stent 1. When the contracting member is contracted in a manner that the inner circumferential surface of the contracting member is brought into intimate contact with the outer circumferential surface of the stent 1, the stent 1 in the expanded state is pressurized toward the radially inner side by the contracting member and is heated to be contracted so that the stent 1 is mounted on the balloon 4b. The contracting member in this case is a contracting portion configured to contract the stent 1 by pressurizing and heating the stent 1 in the expanded state arranged on the inner peripheral side of the mounting jig 7.

### Second Modification of Training Device System

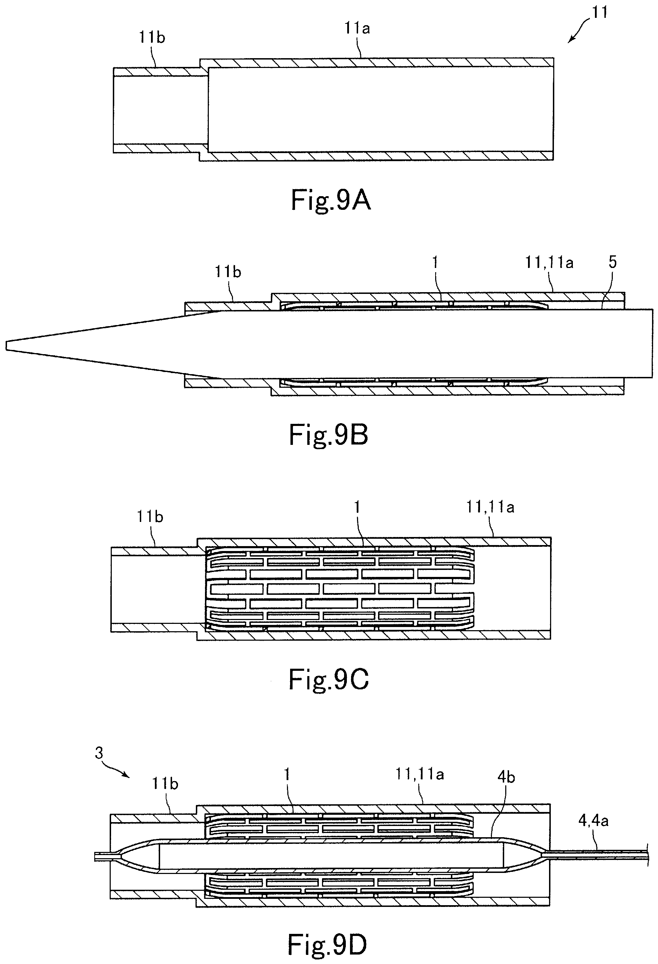

Figs. 9A to 9D are sectional views for illustrating a structure of an expanding jig 11 for expanding the diameter of the stent 1 illustrated in Figs. 1A and 1B.

In the embodiment described above, the training device system 3 may include an expanding jig 11 that is used together with the mandrel 5 when the stent 1 before mounted on the balloon 4b is expanded under a room temperature environment. The expanding jig 11 is formed into a substantially cylinder. The expanding jig 11 includes a cylindrical first cylinder portion 11a constituting one end-side part of the expanding jig 11 and a cylindrical second cylinder portion 11b constituting the other end-side part of the expanding jig 11. The inner diameter and outer diameter of the second cylinder portion 11b are smaller than the inner diameter and outer diameter of the first cylinder portion 11a, respectively.

The first cylinder portion 11a and the second cylinder portion 11b are coaxially arranged. The inner diameter of the first cylinder portion 11a is substantially equal to the outer diameter of the stent 1 in the expanded state expanded by the mandrel 5, or larger than the outer diameter of the stent 1 in the expanded state. The inner diameter of the second cylinder portion 11b is substantially equal to the outer diameter of a large-diameter part other than a distal end-side part of the mandrel 5, or smaller than the outer diameter of the large-diameter part. In other words, the inner diameter of the second cylinder portion 11b is smaller than the outer diameter of the stent 1 in the expanded state expanded by the mandrel 5. Specifically, the inner diameter of the second cylinder portion 11b is sufficiently smaller than the outer diameter of the stent 1 in the expanded state expanded by the mandrel 5 so that the stent 1 in the expanded state expanded by the mandrel 5 is prevented from entering the second cylinder portion 11b. Note that the outer diameter of the first cylinder portion 11a and the outer diameter of the second cylinder portion 11b may be equal to each other.

In this case, as illustrated in Fig. 9B, the stent 1 mounted on the mandrel 5 is inserted into (on the inner circumferential side of) the first cylinder portion 11a from one end side of the expanding jig 11. Specifically, the stent 1 mounted on the mandrel 5 is inserted into the first cylinder portion 11a from one end side of the expanding jig 11 until one end portion of the stent 1 reaches a stepped surface between the first cylinder portion 11a and the second cylinder portion 11b. Next, the mandrel 5 is removed (see Fig. 9C), and the contracted balloon 4b is inserted in the inner circumferential side of the stent 1 (see Fig. 9D). In this case, a linear member such as a wire may be attached to a distal end of the balloon catheter 4. When a linear member is attached to the distal end of the balloon catheter 4, the contracted balloon 4b is easily inserted in the inner circumferential side of the stent 1.

Then, similarly to the embodiment described above, the balloon 4b is expanded so that the stent 1 and the balloon 4b are brought into intimate contact with each other, and then the balloon 4b and the stent 1 are removed from the expanding jig 11. After that, the balloon 4b and the stent 1 are immersed in heated liquid so that the stent 1 is heated and contracted to be mounted on the balloon 4b. In the case where the training device system 3 includes the expanding jig 11 as described above, the stent 1 and the balloon 4b can be brought into intimate contact with each other relatively easily by using the expanding jig 11. Consequently, the stent 1 can be mounted on the balloon 4b relatively easily by using the expanding jig 11. Note that the balloon 4b and the stent 1 may be immersed in heated liquid together with the expanding jig 11. The above-mentioned mounting jig 7 and the expanding jig 11 may be used together.

### Modification of Training Device

While the stent 1 in the embodiment described above is arranged on the inner side of a tubular part in a human body, such as blood vessels, trachea, large intestine, or bile ducts, and used to expand the tubular part from inside, the stent 1 may be used to tangle and retrieve a thrombus formed in a blood vessel. Specifically, the training device 1 to which the present invention is applied may be a stent-type thrombectomy device.

While the stent itself is the training device 1 in the embodiment described above, the training device 1 to which the present invention is applied may be a device that is partially constituted by the stent. For example, the training device 1 may be a stent graft in which an artificial blood vessel is attached to a stent, or may be a stent valve in which a biological valve (valve produced from animal tissue) is attached to a stent. Also in this case, the stent is formed of a shape-memory alloy. The stent is expandable under a room temperature environment, and when heated to a predetermined temperature, the stent is contracted until the inner diameter of the stent becomes smaller than the outer diameter of the balloon 4b in the contracted state, and returns to the original shape thereof.

Also in this case, similarly to the embodiment described above, in the training using the training device 1, the contracted balloon 4b is inserted in the inner circumferential side of the expanded training device 1 (that is, in the inner circumferential side of the stent), and thereafter the training device 1 through which the balloon 4b is inserted in the inner circumferential side thereof is heated to contract the stent. When the stent is contracted so that the training device 1 is mounted on the balloon 4b, the training device 1 mounted on the balloon 4b is inserted inside the three-dimensional model 2, and the balloon 4b is expanded under a room temperature environment to expand the stent so that the training device 1 is placed inside the three-dimensional model 2. Then, the balloon 4b is contracted, and the balloon catheter 4 is removed, so that the training device 1 is placed inside the three-dimensional model 2.

When the training using the training device 1 is finished, the training device 1 placed inside the three-dimensional model 2 is heated to a predetermined temperature so that the stent is contracted to return to its original state. When the stent returns to its original state, the training device 1 placed inside the three-dimensional model 2 is contracted to enable the training device 1 to be removed from inside the three-dimensional model 2. Thus, the training device 1 is removed and retrieved from inside the three-dimensional model 2. Also in this case, similarly to the embodiment described above, the retrieved training device 1 can be repeatedly used for training in which the training device 1 is inserted and placed inside the three-dimensional model 2. Consequently, the cost of training in which the training device 1 is inserted and placed inside the three-dimensional model 2 can be reduced.

The training device 1 to which the present invention is applied may be a device to be mounted on the balloon 4b of the balloon catheter 4 for use, other than a stent, a stent-type thrombectomy device, a stent graft, and a stent valve.

### Other Embodiments

While the stent 1 in the expanded state, which is in intimate contact with the outer circumferential surface of the balloon 4b in the expanded state, is heated and contracted in the liquid in the embodiment described above, the stent 1 in the expanded state in which the contracted balloon 4b has been inserted in the inner circumferential side thereof (see Fig. 3B) may be heated and contracted in the liquid. In order to mount the stent 1 on an intended position on the balloon 4b, it is preferred that the stent 1 in the expanded state in intimate contact with the outer circumferential surface of the balloon 4b in the expanded state is heated and contracted in the liquid as in the embodiment described above.

While the balloon 4b and the stent 1 are immersed in the liquid and the stent 1 is heated and contracted in the liquid in the embodiment described above, heated liquid, such as water, normal saline, or a contrast agent, may be poured into the balloon catheter 4 so that the stent 1 is heated and contracted to be mounted on the balloon 4b without being heated from outside the stent 1. In this case, the contracted balloon 4b is inserted in the inner circumferential side of the stent 1 in the expanded state, and heated liquid, such as water, normal saline, or a contrast agent, is poured into the balloon catheter 4 with the indeflator. After the stent 1 and the balloon 4b are sufficiently brought into intimate contact with each other, the balloon 4b is contracted. The stent 1 is heated by the liquid in the balloon 4b, and hence when the balloon 4b is contracted, the stent 1 is also contracted to be mounted on the balloon 4b.

While the stent 1 is used for training in which the stent 1 is inserted and placed in the tube portion 2a of the three-dimensional model 2 in the embodiment described above, the stent 1 may be used for property evaluation test of the stent 1.

## Claims

1. A training device (1) to be inserted and placed inside a three-dimensional model (2) of a biological organ manufactured for training, under a state in which the training device is mounted on a balloon (4b) of a balloon catheter (4), wherein
the training device (1) is a cylindrical stent formed of a shape-memory alloy or comprises a cylindrical stent formed of a shape-memory alloy,
the stent is expandable under a room temperature environment, and
the stent is contracted when heated to a predetermined temperature higher than room temperature, until an inner diameter of the stent becomes smaller than an outer diameter of the balloon (4b) in a contracted state, and returns to an original shape thereof,
and further wherein the stent has a retaining portion (1a, 1b) configured to prevent the stent in the contracted state from falling off from the balloon (4b) in the contracted state.

2. The training device (1) according to claim 1, wherein the retaining portion (1a; 1b) is at least one of a rough surface portion formed on an inner circumferential surface of the stent, a protruding portion (1b) that protrudes to an inner circumferential side of the stent, and a small-diameter portion (1a) that is formed at an end portion of the stent and has an inner diameter smaller than at a central part of the stent.

3. The training device (1) according to claim 1 or 2, wherein the stent has a hook portion (1c) configured to catch a distal end portion of a removal instrument for removing the training device (1) placed inside the three-dimensional model (2).

4. A training device system (3) comprising:
the training device (1) according to any one of claims 1 to 3, and
the balloon catheter (4).

5. The training device system (3) according to claim 4, wherein
the balloon (4b) has a protruding portion (4c) formed or fixed on an outer circumferential surface thereof, the protruding portion being arranged on both sides or one side of the stent and configured to prevent the stent in a contracted state from falling off from the balloon (4b) in a contracted state.

6. The training device system (3) according to claim 4 or 5, further comprising a cylindrical mounting jig (7) for mounting the training device (1) on the balloon (4b), wherein
the stent in the expanded state is arrangeable on an inner peripheral side of the mounting jig (7),
the mounting jig (7) includes a contracting portion (9) configured to contract the stent by performing at least one of pressurization and heating of the stent in the expanded state arranged on the inner peripheral side of the mounting jig (7), and
when the contracting portion (9) contracts the stent under a state in which the stent in the expanded state is arranged on the inner peripheral side of the mounting jig (7) and the balloon (4b) in the contracted state is inserted in an inner circumferential side of the stent, the training device (1) is mounted on the balloon (4b).

7. The training device system (3) according to any one of claims 4 to 6, further comprising:
a mandrel (5) to be inserted in an inner circumferential side of the stent in order to expand the stent under a room temperature environment; and
a substantially cylindrical expanding jig (11) that is used together with the mandrel (5) in order to expand the stent under a room temperature environment and in which the stent is arranged on an inner circumferential side thereof, wherein
the expanding jig (11) includes:
a cylindrical first cylinder portion (11a) that constitutes one end-side part of the expanding jig (11); and
a cylindrical second cylinder portion (11b) that constitutes another end-side part of the expanding jig (11) and that is arranged coaxially with the first cylinder portion (11a),
the first cylinder portion (11a) has an inner diameter that is substantially equal to an outer diameter of the stent expanded by the mandrel (5), or larger than the outer diameter of the stent expanded by the mandrel(5), the second cylinder portion (11b) has an inner diameter that is smaller than the outer diameter of the stent expanded by the mandrel (5), and
the stent mounted on the mandrel (5) is inserted in an inner circumferential side of the first cylinder portion (11a) from one end side of the expanding jig (11).

## Patentansprüche

1. Trainingsvorrichtung (1) zum Einführen und Platzieren in einem dreidimensionalen Modell (2) eines zum Training hergestellten biologischen Organs in einem Zustand, in dem die Trainingsvorrichtung auf einem Ballon (4b) eines Ballonkatheters (4) angebracht ist,
wobei die Trainingsvorrichtung (1) ein zylindrischer Stent ist, der aus einer Formgedächtnislegierung gebildet ist, oder einen aus einer Formgedächtnislegierung gebildeten zylindrischen Stent aufweist,
wobei der Stent in einer Umgebung mit Raumtemperatur aufweitbar ist, und wobei der Stent kontrahiert wird, wenn er auf eine vorbestimmte Temperatur erwärmt wird, die höher als die Raumtemperatur ist, bis ein Innendurchmesser des Stents kleiner wird als ein Außendurchmesser des Ballons (4b) in einem kontrahierten Zustand, sowie in seine ursprüngliche Form zurückkehrt, und wobei ferner der Stent einen Haltebereich (1a, 1b) aufweist, der dazu ausgebildet ist, zu verhindern, dass der Stent im kontrahierten Zustand von dem Ballon (4b) im kontrahierten Zustand herunterfällt.

2. Trainingsvorrichtung (1) nach Anspruch 1,
wobei der Haltebereich (1a; 1b) mindestens eines von einem rauen Oberflächenbereich, der auf einer Innenumfangsfläche des Stents gebildet ist, einem vorstehenden Bereich (1b), der auf eine Innenumfangsseite des Stents hervorsteht, und einem Bereich (1a) mit kleinem Durchmesser ist, der an einem Endbereich des Stents gebildet ist und einen Innendurchmesser aufweist, der kleiner als an einem zentralen Teil des Stents ist.

3. Trainingsvorrichtung (1) nach Anspruch 1 oder 2,
wobei der Stent einen Hakenbereich (1c) aufweist, der dazu ausgebildet ist, einen distalen Endbereich eines Entfernungsinstruments aufzunehmen, um die im Inneren des dreidimensionalen Modells (2) platzierte Trainingsvorrichtung (1) zu entfernen.

4. Trainingsvorrichtungssystem (3), aufweisend:
die Trainingsvorrichtung nach einem der Ansprüche 1 bis 3 und den Ballonkatheter (4).

5. Trainingsvorrichtungssystem (3) nach Anspruch 4,
wobei der Ballon (4b) einen hervorstehenden Bereich (4c) aufweist, der an einer Außenumfangsfläche desselben gebildet oder festgelegt ist, wobei der hervorstehende Bereich auf beiden Seiten oder auf einer Seite des Stents angeordnet und dazu ausgebildet ist, zu verhindern, dass der Stent in einem kontrahieren Zustand von dem Ballon (4b) in einem kontrahierten Zustand herunterfällt.

6. Trainingsvorrichtungssystem (3) nach Anspruch 4 oder 5,
weiterhin aufweisend eine zylindrische Montagevorrichtung (7) zum Anbringen der Trainingsvorrichtung (1) auf dem Ballon (4b),
wobei sich der Stent im aufgeweiteten Zustand auf einer Innenumfangsseite der Montagevorrichtung (7) anordnen lässt,
wobei die Montagevorrichtung (7) einen Kontraktionsbereich (9) aufweist, der zum Kontrahieren des Stents ausgebildet ist, indem mindestens eines von einer Druckbeaufschlagung und einer Erwärmung des auf der Innenumfangsseite der Montagevorrichtung (7) angeordneten Stents in dem aufgeweiteten Zustand ausgeführt wird, und
wobei dann, wenn der Kontraktionsbereich (9) den Stent in einem Zustand kontrahiert, in dem der Stent in dem aufgeweiteten Zustand auf der Innenumfangsseite der Montagevorrichtung (7) angeordnet ist und der Ballon (4b) im kontrahierten Zustand in eine Innenumfangsseite des Stents eingeführt ist, die Trainingsvorrichtung (1) an dem Ballon (4b) angebracht ist.

7. Trainingsvorrichtungssystem (3) nach einem der Ansprüche 4 bis 6, weiterhin aufweisend:
einen Dorn (5) zum Einführen in eine Innenumfangsseite des Stents, um den Stent in einer Umgebung mit Raumtemperatur aufzuweiten; und
eine im Wesentlichen zylindrische Aufweitvorrichtung (11), die zusammen mit dem Dorn (5) verwendet wird, um den Stent in einer Umgebung mit Raumtemperatur aufzuweiten, und in der der Stent auf einer Innenumfangsseite derselben angeordnet ist,
wobei die Aufweitvorrichtung (11) Folgendes aufweist:
einen zylindrischen ersten Zylinderbereich (11a), der einen endseitigen Bereich der Aufweitvorrichtung (11) bildet; und
einen zylindrischen zweiten Zylinderbereich (11b), der einen weiteren endseitigen Bereich der Aufweitvorrichtung (11) bildet und der koaxial mit dem ersten Zylinderbereich (11a) angeordnet ist,
wobei der erste Zylinderbereich (11a) einen Innendurchmesser aufweist, der im Wesentlichen gleich einem Außendurchmesser des durch den Dorn (5) aufgeweiteten Stents ist oder größer als der Außendurchmesser des durch den Dorn (5) aufgeweiteten Stents ist,
wobei der zweite Zylinderbereich (11b) einen Innendurchmesser aufweist, der kleiner als der Außendurchmesser des durch den Dorn (5) aufgeweiteten Stents ist, und
wobei der auf dem Dorn (5) angebrachte Stent von einer Endseite der Aufweitvorrichtung (11) in eine Innenumfangsseite des ersten Zylinderbereichs (11a) eingeführt wird.

## Revendications

1. Dispositif d'entraînement (1) à introduire et à placer à l'intérieur d'un modèle tridimensionnel (2) d'un organe biologique fabriqué pour l'entraînement, dans un état où le dispositif d'entraînement est monté sur un ballonnet (4b) d'un cathéter à ballonnet (4), dans lequel
le dispositif d'entraînement (1) est une endoprothèse vasculaire cylindrique formée d'un alliage à mémoire de forme, ou comprend une endoprothèse vasculaire cylindrique formée d'un alliage à mémoire de forme, et
l'endoprothèse vasculaire peut s'agrandir dans un environnement à température ambiante, et
l'endoprothèse vasculaire peut se contracter lorsque chauffée à une température prédéterminée supérieure à la température ambiante, jusqu'à ce qu'un diamètre intérieur de l'endoprothèse vasculaire devienne plus petit qu'un diamètre extérieur du ballonnet (4b) dans un état contracté, et revienne à la forme originale de celle-ci, et
dans lequel en outre, l'endoprothèse vasculaire présente une portion de retenue (1a, 1b) configurée pour empêcher l'endoprothèse vasculaire dans l'état contracté de tomber du ballonnet (4b) dans l'état contracté.

2. Dispositif d'entraînement (1) selon la revendication 1, dans lequel la portion de retenue (1a; 1b) est au moins une portion parmi une portion de surface grossière formée sur une surface circonférentielle intérieure de l'endoprothèse vasculaire, une portion saillante (1b) faisant saillie vers un côté circonférentiel intérieur de l'endoprothèse vasculaire, et une portion de petit diamètre (1a), laquelle est formée sur une portion d'extrémité de l'endoprothèse vasculaire et présente un diamètre intérieur plus petit que sur une partie centrale de l'endoprothèse vasculaire.

3. Dispositif d'entraînement (1) selon la revendication 1 ou 2, dans lequel l'endoprothèse vasculaire présente une portion à crochet (1c) configurée pour attraper une portion d'extrémité distale d'un instrument de retrait pour retirer le dispositif d'entraînement (1) placé à l'intérieur du modèle tridimensionnel (2).

4. Système de dispositif d'entraînement (3), comprenant :
le dispositif d'entraînement (1) selon l'une quelconque des revendications 1 à 3, et le cathéter à ballonnet (4).

5. Système de dispositif d'entraînement (3) selon la revendication 4, dans lequel
le ballonnet (4b) présente une portion saillante (4c) formée ou fixée sur une surface circonférentielle extérieure de celui-ci, la partie saillante étant agencée des deux côtés, ou sur un seul côté de l'endoprothèse vasculaire, et configurée pour empêcher l'endoprothèse vasculaire dans l'état contracté de tomber du ballonnet (4b) dans l'état contracté.

6. Système de dispositif d'entraînement (3) selon la revendication 4 ou 5, comprenant en outre un moyen de montage (7) pour disposer le dispositif d'entraînement (1) sur le ballonnet (4b),
dans lequel
l'endoprothèse vasculaire à l'état agrandi peut être agencée sur un côté périphérique intérieur du moyen de montage (7) ;
le moyen de montage (7) inclut une portion de contraction (9), configurée pour contracter l'endoprothèse vasculaire en réalisant au moins une pressurisation et un chauffage de l'endoprothèse vasculaire à l'état agrandi agencée sur le côté périphérique intérieur du moyen de montage (7), et
lorsque la portion de contraction (9) contracte l'endoprothèse vasculaire dans un état où l'endoprothèse vasculaire dans l'état agrandi est agencée sur le côté périphérique intérieur du moyen de montage (7) et que le ballonnet (4b) dans l'état contracté est introduit dans un côté circonférentiel intérieur de l'endoprothèse vasculaire, le dispositif d'entraînement (1) est monté sur le ballonnet (4b).

7. Système de dispositif (3) selon l'une quelconque des revendications 4 à 6, comprenant en outre :
un mandrin (5) à introduire dans un côté circonférentiel intérieur de l'endoprothèse vasculaire afin d'agrandir l'endoprothèse vasculaire dans un environnement à température ambiante, et
un moyen d'agrandissement en grande partie cylindrique (11), lequel est utilisé conjointement au mandrin (5) afin d'agrandir l'endoprothèse vasculaire dans un environnement à température ambiante, et où l'endoprothèse vasculaire est agencée sur un côté circonférentiel intérieur, dans lequel
le moyen d'agrandissement (11) inclut :
une première portion cylindrique formant cylindre (11a), laquelle constitue une partie côté extrémité du moyen d'agrandissement (11), et
une seconde portion cylindrique formant cylindre (11b), laquelle constitue une autre partie côté extrémité du moyen d'agrandissement (11), et laquelle est agencée de manière coaxiale avec la première portion formant cylindre (11a) ;
la première portion formant cylindre (11a) présente un diamètre intérieur en grande partie égal à un diamètre extérieur de l'endoprothèse vasculaire agrandie par le mandrin (5), ou supérieur au diamètre extérieur de l'endoprothèse vasculaire agrandie par le mandrin (5) ;
la seconde portion formant cylindre (11b) présente un diamètre intérieur plus petit que diamètre extérieur de l'endoprothèse vasculaire agrandie par le mandrin (5), et l'endoprothèse vasculaire montée sur le mandrin (5) est introduite dans un côté circonférentiel intérieur de la première portion formant cylindre (11a) à partir d'un côté extrémité du moyen d'agrandissement (11).
